# EUROPÄISCHE PATENTSCHRIFT

(11) **EP 0 563 998 B1**
(45) Veröffentlichungstag und Bekanntmachung des Hinweises auf die Patenterteilung: **25.11.1998**
(21) Anmeldenummer: 93105538.8
(22) Anmeldetag: 02.04.1993
(51) Int. Cl.: G01N 21/64

(54) **Verfahren zur Erfassung von Biomolekülen, toxischen Substanzen, Polymeren und pharmazeutischen Wirkstoffen mittels zeitaufgelöster Laserspektroskopie**
A process for detecting biomolecules, toxic substances, polymers and pharmaceutical substances by time-resolved laser spectroscopy
Procédé de détection de molecules biologiques, de substances toxiques, de polymèrs de substances pharmaceutiques par spectroscopic laser à résolution temporelle

(30) Priorität: 02.04.1992 DE 4210970
(43) Veröffentlichungstag der Anmeldung: 06.10.1993
(73) Patentinhaber: Sauer, Markus, D-75175 Pforzheim (DE); DREXHAGE, Karl-Heinz, Prof. Dr., D-57068 Siegen (DE); Han, Kyung-Tae, D-71543 Wüstenrot (DE); Köllner, Malte, D-69115 Heidelberg (DE); Schulz, Andreas, D-67117 Limburgerhof (DE); Seeger, Stefan, Dr., 69121 Heidelberg (DE); Seidel, Claus, D-69120 Heidelberg (DE); Wolfrum, Jürgen, Prof. Dr., D-37124 Rosdorf (DE)
(72) Erfinder: Sauer, Markus, D-75175 Pforzheim (DE); DREXHAGE, Karl-Heinz, Prof. Dr., D-57068 Siegen (DE); Han, Kyung-Tae, D-71543 Wüstenrot (DE); Köllner, Malte, D-69115 Heidelberg (DE); Schulz, Andreas, D-67117 Limburgerhof (DE); Seeger, Stefan, Dr., 69121 Heidelberg (DE); Seidel, Claus, D-69120 Heidelberg (DE); Wolfrum, Jürgen, Prof. Dr., D-37124 Rosdorf (DE)
(74) Vertreter: Schmid, Rudolf, Dipl.-Ing., Patentanwalt

(56) Entgegenhaltungen:
- EP-A- 0 263 037
- EP-A- 0 296 136
- DE-A- 4 104 014
- OPTICAL ENGINEERING Bd. 14, Nr. 6, Dezember 1985, BELLINGHAM US Seiten 1042 - 1044 H.SCHNECKENBURGER 'Time resolved microfluorescence in biomedical diagnosis'
- TRAC, TRENDS IN ANALYTICAL CHEMISTRY Bd. 8, Nr. 1, Januar 1989, CAMBRIDGE GB Seiten 29 - 34 G.J.BLANCHARD 'Applications of picosecond spectroscopy to analytical chemistry'

## Beschreibung

Die Erfindung betrifft ein Verfahren zur simultanen optischen qualitativen und quantitativen Erfassung von verschiedenen mit Fluorochromon oder Fluorogenen markierten Molekülen eines Gemisches mittels Laserspektroskopie gemäß dem Oberbegriff des Anspruchs 1, wie aus EP 0 263 037 A2 bekannt. Die zu bestimmenden Moleküle können hierbei Biomoleküle wie Nukleinsäuren, Proteine, Peptide, Hormone, Antigene, Haptene, Antikörper, toxische Substanzen wie Umweltgifte, Pestizide, Polymere oder pharmazeutische Wirkstoffe, wie Alkaloide, sein.

Der hochempfindliche Nachweis dieser Moleküle ist in vielen Bereichen von Bedeutung. Er eröffnet einen wichtigen Zugang für das Verständnis der Lebensvorgänge und der Wirkungsweise von Pharmazeutika in Zellen. Die quantitative Erfassung von körpereigenen Molekülen (z. B. Hormone), die nur in minimalen Konzentrationen vorliegen, spielt eine wesentliche Rolle bei der Aufdeckung und Behandlung vieler Krankheiten. Auch die Umweltanalytik erfordert immer empfindlichere Nachweismethoden zur Bestimmung von Umweltgiften. Daneben kann das Verfahren auch in technischen Prozessen, beispielsweise zur Überwachung der Produktion in der Polymerherstellung, verwendet werden.

Zur qualitativen und quantitativen Erfassung dieser Moleküle sind bereits Fluoreszenztechniken entwickelt worden, die sich gegenüber der radioaktiven Markierung oder der Verwendung von Enzymen durch ihre Schnelligkeit, ihre stabile Kopplungsmöglichkeit und ihre extreme Empfindlichkeit auszeichnen. Fluoreszenz-Labeling ist eine geeignete Methode für analytische Bestimmungen, gerade wenn die nachzuweisende Substanz selbst fluoresziert, ihre Anregungs- und Emissionswellenlänge jedoch in einem Spektralbereich liegen, in dem die Detektion schwierig ist. Die Einfachheit, Selektivität und Empfindlichkeit machte die Fluoreszenztechnik zu einer bevorzugten Methode für analytische Bestimmungen (Anal.Chem. 63 (1991) 321). In den letzten Jahren wurden entscheidende Fortschritte durch den Einsatz von verschiedenen Lasersystemen in der Fluoreszenzanalytik gemacht. Die bisher meist verwendeten Fluorochrome sind aromatische, polycyclische Kohlenwasserstoffe, aber auch einige Proteine, anorganische Ionen, Kristalle und Chelate finden ihre Anwendung (Applied Fluorescence Technology Vol. I(1989)4). Im Übersichtsartikel von F.V. Bright sind die meist verwendeten Fluoreszenzlabels zusammengestellt (Bright, F. V., Anal. Chem. 60 (1988) 1031A). Ein weiterer Vorteil der optischen Markierung in der Fluoreszenztechnik ist die meist verwendete stabile und selektive kovalente Kopplung an definierte Substrate. Die Empfindlichkeit der Methode wird hauptsächlich durch die Untergrundfluoreszenz limitiert, so daß zur hochempfindlichen Detektion hauptsächlich Farbstoffe, die im nahen IR- Bereich absorbieren, Verwendung finden. In der nahen IR- Region existieren nur wenige Moleküle, die eine signifikante Absorption oder Emission besitzen (Rodd's Chemistry of Carbon Compounds; 2nd ed.: Coeffy, S., Ed.: Elsevier: New York, 1977).

Ein limitierender Faktor der Empfindlichkeit stellt das instantan, also sofort auftretende Streulicht der Probe dar. Die meisten Verfahren arbeiten mit der stationären Fluoreszenzdetektion, so daß zur Unterdrückung des Streulichtes eine Filtertechnik angewandt werden muß, die die Photonenausbeute, also die Ausbeute der induzierten Strahlung reduziert und damit die Empfindlichkeit der Methode verschlechtert (Anal. Chem. 63 (1991) 2835-2841). Der Hauptbestandteil des Streulichtes wird meist durch die Ramanstreuung des Lösungsmittels hervorgerufen. Wird die Fluoreszenz gegenüber der Anregung zeitlich verzögert detektiert, so können selbst einzelne Farbstoffmoleküle nachgewiesen werden (Chem. Phys. Lett. 174 (1990) 553). Die Verfahren benutzen verschiedene Farbstoffe für verschiedene, nachzuweisende Moleküle. Da sich die angekoppelten Farbstoffe in ihren Absorptionswellenlängen unterscheiden, werden für ihre Bestimmung unterschiedliche Laserwellenlängen benötigt (Nucleic Acid Res. 18 (1990) 1415-1419). Normalerweise muß, wenn verschiedene Stoffe in einer Mischung vorliegen, folgender Kompromiß gemacht werden: Entweder man benutzt verschiedene Laserwellenlängen für jedes Absorptionsmaximum der verwendeten Farbstoffe, wozu meist verschiedene Lasersysteme gebraucht werden, oder man arbeitet nur bei einer Anregungswellenlänge, erniedrigt hierbei jedoch die Anregungseffizienz, da die eingesetzten Farbstoffe nicht am Absorptionsmaximum angeregt werden.

Die aus der Literaturstelle "Trend in "Analytical Chemistry", Vol. 8, No. 1, 1989, S. 29 - 34 bekannten Farbstoff Oxazin und Resorufin, mit dem gemeinsamen Grundgerüst, können nicht als Fluoreszenzfarbstoffmolekül im eigentlichen Sinne angesehen werden. Erst durch die Substituenten "NH₂" bzw. "0", welche als elektronenziehenden bzw. elektronenspendenden Gruppen das II-Elektronensystem entsprechend beeinflussen, wird das Grundgerüst zum Farbstoff.

Aus der eingangs genannten EP 0 263 037 A2 sowie der Literaturstelle "Optical Engineering" Bd. 14, Nr. 6, Dez. 1985, sind Verfahren bekannt, beim denen verschiedene, mit Farbstoffen oder Farbstoffkonjugaten markierten Moleküle, simultan optisch, qualitativ erfaßt werden können, wobei die Moleküle in einem Lasersystem mit kurzen Lichtimpulsen angeregt werden und das entstandene Fluoreszenzlicht mit zeitlicher Auflösung registriert wird.

Der Erfindung liegt die Aufgabe zugrunde, ein Verfahren zur simultanen optischen qualitativen und quantitativen Erfassung von verschiedenen mit Fluorochromen oder Fluorogenen markierten Molekülen eines Gemisches bereitzustellen, bei dem für die effiziente Anregung der Makierungsfarbstoffe eine einzige Lichtquelle und zur Detektion des Fluoreszenzsignals nur ein einziges Filtersystem benötigt wird.

Die Lösung dieser Aufgabe erfolgt bei dem Verfahren nach dem Oberbegriff des Anspruchs 1 durch die in seinem kennzeichnenden Teil angegebenen Merkmale.

Die bei dem Verfahren verwendeten Farbstoffe oder Farbstoffkonjugate besitzen also die gleiche Anregungswellenlänge, überlappende Emissionsbanden, aber unterschiedliche Fluoreszenzlebensdauern und Quantenausbeuten. Farbstoffe, die diese Eigenschaften erfüllen, werden im folgenden als Multiplexfarbstoffe bezeichnet. Durch die Detektion des zeitlichen Verlaufs der Fluoreszenzintensität (Fluoreszenzlebensdauer) kann auf die Filtertechnik verzichtet und das Streulicht zeitlich unterdrückt werden, was zu einer Empfindlichkeitssteigerung gegenüber den bisher verwendeten Verfahren führt.

Farbstoffe oder durch ein oder mehrere Farbstoffmoleküle markierte Moleküle (im Folgenden als Farbstoffkonjugate bezeichnet werden) an Biomoleküle, toxische Substanzen, Polymere oder pharmazeutische Wirkstoffe gekoppelt und über die vom markierten Molekül unbeeinflußte, farbstoffspezifische Fluoreszenzlebensdauer charakterisiert. Die so markierten Moleküle können spezifisch an das nachzuweisende Molekül gekoppelt werden (z.B. ein markierter Antikörper für das nachzuweisende Antigen). Die Methode erlaubt eine Mehrfachnutzung ("Multiplexen") von Farbstoffen: Die verwendeten Fluoreszenzfarbstoffe besitzen, durch spezifisch wirkende Substituenten am Farbstoffgrundgerüst, verschiedene Fluoreszenzlebensdauern bei annähernd gleicher Absorptions- und Emissionswellenlänge. Spezifisch wirkende Substituenten am Farbstoffgrundgerüst bedeutet, daß durch zusätzliche Chromophore Gruppen am Farbstoffgrundgerüst dessen mittlere Verweilzeit im ersten angeregten elektronischen Zustand beeinflußt werden kann.

Als Anregungsquelle können alle Lasersysteme die Pulse im Nanosekundenbereich oder kürzer (ps, fs) liefern, wie Festkörperlaser, Farbstofflaser, Ionenlaser, Gaslaser, chemische Laser, vorzugsweise jedoch Diodenlaser verwendet werden. Die Detektion des Fluoreszenzsignals soll vorzugsweise mit zeitaufgelöster Einzelphotonenzählung erfolgen.

Charakterisierung der Moleküle bedeutet, daß das zu bestimmende Molekül durch direkte Ankopplung eines Fluoreszenzfarbstoffes oder indirekt durch die Ankopplung eines spezifischen, markierten Moleküls (Farbstoffkonjugat) aufgrund von optischen Eigenschaften identifiziert und detektiert wird. Dabei wird die Fluoreszenzlebensdauer und die Fluoreszenzemissionswellenlänge des Farbstoffes genutzt. Zur Charakterisierung sind nur solche Farbstoffe geeignet, die sich nicht durch die Umgebung "quenchen" lassen, d. h. es treten keine Wechselwirkungen zwischen dem Farbstoff oder Farbstoffkonjugat und dem zu bestimmenden Molekül auf, die Fluoreszenzlebensdauer bleibt unbeeinflußt.

Die erreichbare Empfindlichkeit der Fluoreszenztechnik wird hauptsächlich durch die Untergrundfluoreszenz während der Messung und durch das Signal/Rausch-Verhältnis bestimmt. Um Substanzmengen im Atto- (10⁻¹⁸) und Zeptomolbereich (10⁻²¹) noch sicher nachweisen zu können, muß die Fluoreszenzprobe einen großen Extinktionskoeffizienten bei der Anregungswellenlänge, eine hohe Photostabilität und eine ausreichende Quantenausbeute besitzen. Als Farbstoffe eignen sich Fluoreszenzfarbstoffe wie Coumarine, Flavine, Fluoresceine, Carbocyanine, Phenoxazone, Carbazine, Oxazine und insbesondere Rhodamine, vorzugsweise jedoch solche Farbstoffe, die im fernen UV/vis - bzw. nahen IR - Bereich (600-1000 nm) absorbieren und fluoreszieren.

Die Markierung eines Moleküls mit einem Fluoreszenzfarbstoff erfordert eine chemisch aktivierte Kopplungsgruppe am Farbstoffgerüst. Solche Gruppen sind beispielsweise Thiocyanate, Isothiocyanate, N-Hydroxysuccinimidester, α-Halogenacetyl-, Maleinimid- und Allylbromidgruppen. Aus sterischen Gründen kann der Einbau eines spacers erforderlich sein, der einen definierten Abstand zwischen dem zu markierenden Molekül und dem Farbstoffmolekül sichert. Die Moleküle werden mit den verschiedenen Farbstoffkonjugaten bevorzugt so markiert, daß Substanz A nur mit Farbstoffkonjugat F_{A}, B nur mit F_{B}, C nur mit F_{C}, usw. reagiert. Die zu markierenden Moleküle können mit den entsprechend chemisch aktivierten Fluoreszenzfarbstoffen beispielsweise über eine Amin-, Thiol- oder Hydroxylfunktion kovalent markiert werden (Photochem. Photobiol. 52 (1990) 431). Chemische Aktivierung bedeutet, daß der Farbstoff durch die oben erwähnten Gruppen in seinen Reaktionseigenschaften so modifiziert wird, daß er spezifisch mit den zu markierenden Molekülen reagiert.

Es werden außer der Information, die aus der Fluoreszenzwellenlänge erhalten werden kann, die verschiedenen Fluoreszenzlebensdauern (Multiplexfarbstoffe), ausgenützt so daß die Methode eine erhebliche Zeiteinsparung durch die gleichzeitige Bestimmung unterschiedlicher Moleküle, die im Gemisch vorliegen, ermöglicht.

Durch den Einsatz von Multiplexfarbstoffen wird es ermöglicht, beispielsweise in der DNA-Sequenzierung, nur eine Trennbahn für alle Basen zu verwenden (DNA-Sequenzierung nach einem 4-Farbstoff-1-Bahn-Prinzip). Die Multiplexfarbstoffe besitzen, da sie sich nur im Substitutionsmuster am Farbstoffgrundgerüst unterscheiden die gleiche Mobilität im Elektrophoresegel, so daß eine Korrektur der Laufzeiten nicht nötig ist. Ferner kann durch die Verwendung von Multiplexfarbstoffen, die sich in ihren Absorptionseigenschaften nicht oder nur geringfügig unterscheiden, die gleiche Lichtanregungsquelle zur parallelen Anregung mehrerer markierter Moleküle benutzt werden.

Ein weiterer Vorteil des beschriebenen Verfahrens ist die Tatsache, daß die Fluoreszenzlebensdauern der Farbstoffe nicht exakt bestimmt, sondern nur wiedererkannt und von den anderen Fluoreszenzlebensdauern unterschieden werden müssen. Zur qualitativen und quantitativen Erfassung der markierten Moleküle sind bei dieser Methode weniger Fluoreszenzphotonen notwendig, so daß das Verfahren bei der Verwendung von hochrepetierenden Laserdioden für die Bestimmung in schnellen Trennsystemen, beispielsweise in der Kapillargelelektrophorese, sehr gut geeignet ist.

Durch die optische Charakterisierung der nachzuweisenden Moleküle über die Fluoreszenziebensdauer ist die verwendete Technik hochempfindlich und erlaubt den Nachweis geringster Molekülmengen (Atto- und Zeptomolbereich). Wird zur Fluoreszenzdetektion die Abklingzeit der Fluoreszenz (Fluoreszenzlebensdauer) benutzt, so kann das instantan auftretende Streulicht von der zeitlich verzögerten Fluoreszenz diskriminiert werden. Dies bedeutet eine wesentliche Empfindlichkeitssteigerung gegenüber der stationären Fluoreszenzmessung, da auf die Filtertechnik, durch die die Photonenausbeute verringert wird, verzichtet werden kann.

Die Erfindung wird im Folgenden anhand von einigen Beispielen näher erläutert.
1.) Anhand der Coumarinfarbstoffe soll das Prinzip des Multiplexen von Farbstoffen gezeigt werden. Durch geringe Veränderungen am Substitutionsmuster des Farbstoffgrundgerüstes wird die Absorptions- und Emissionswellenlänge der Farbstoffe in Wasser nur gering, die Fluoreszenzlebensdauer hingegen stark beeinflußt. Die Fluoreszenzlebensdauern werden mit einer Einzelphotonenzählapparatur bestimmt. Als Anregungsquelle dient eine mit Stickstoff gefüllte Hochspannungsblitzlampe, die Lichtblitze im Nanosekundenbereich (4-7 ns) liefert. Die käuflich erwerblichen Farbstoffe werden mittels Absorptionsspektroskopie für die Messungen auf eine optische Dichte von 0.06 eingestellt. Als Lösungsmittel für die Coumarinfarbstoffe wird Wasser verwendet. Die Proben werden in einer Halbmikroquarzküvette mit Thermostatisierung bei 20°C vermessen. Alle hier angegebenen Coumarine werden bei 337 nm (Abs.) angeregt. Die Detektion der induzierten Emission (Em.) erfolgt im 90° Winkel zur Anregung bei 420 nm.

| | | |
|---|---|---|
| 7-Amino-4-methylcoumarin | τ = 5.0 ns | Abs. λₘₐₓ = 340 nm; Em. λₘₐₓ = 443 nm |
| 6,7-Dimethoxy-4-trifluormethylcoumarin | τ = 4.1 ns | Abs. λₘₐₓ = 330 nm; Em. λₘₐₓ = 419 nm |
| 7-Methoxy-3-cyanocoumarin | τ = 2.5 ns | Abs. λₘₐₓ = 355 nm; Em. λₘₐₓ = 410 nm |
| 7-Methoxy-3-carbonsäureethylester | τ = 1.8 ns | Abs. λₘₐₓ = 350 nm; Em. λₘₐₓ = 408 nm |

2.) Die zwei Rhodamin-Multiplexfarbstoffe zeigen, daß selbst Fluoreszenzfarbstoffe mit den gleichen Substituenten und übereinstimmendem Molekulargewicht durch einen kleinen Unterschied im Substitutionsmuster unterschiedliche Fluoreszenzlebensdauern zeigen. Durch das gleiche Molekulargewicht der Farbstoffe sind sie für den Einsatz bei elektrophoretischen Auftrennungen ideal geeignet, da die normal notwendigen Korrekturen durch die unterschiedlichen Molekulargewichte der verwendeten Farbstoffe hinfällig werden. Die Messungen werden entsprechend den Fluoreszenzlebensdauerbestimmungen der Coumarinderivate durchgeführt. Die Rhodamine werden bei 316 nm in Ethanol angeregt und bei der jeweiligen Maximumemissionswellenlänge detektiert.

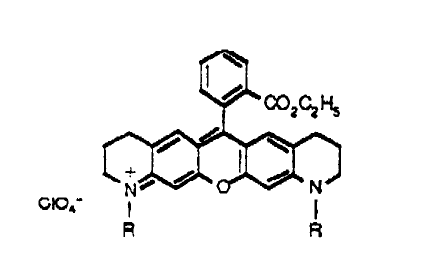

| | |
|---|---|
| DR 271 (R = -CH₂- o -Nitrophenyl) in Ethanol | τ = 2.6 ns |
| | Abs. λₘₐₓ = 553 nm; Em. λₘₐₓ = 580 nm |
| DR 273 (R = -CH₂- m -Nitrophenyl) in Ethanol | τ= 3.7 ns |
| | Abs. λₘₐₓ = 556 nm; Em. λₘₐₓ = 582 nm |

## Patentansprüche

1. Verfahren zur simultanen optischen. qualitativen und quantitativen Erfassung von verschiedenen mit Farbstoffen oder Farbstoffkonjugaten markierten Molekülen eines Gemisches, welche in einem Lasersystem mit kurzen Lichtimpulsen zur Fluoreszenz angeregt werden und wobei das abgegebene Fluoreszenzlicht mit zeitlicher Auflösung registriert und ausgewertet wird,
dadurch gekennzeichnet,
daß als Fluoreszenzfarbstoffe Farbstoffe mit spezifisch wirkenden Substituenten am selben Farßftoffgrundgerüst eingesetzt werden, wodurch die Fluoreszenzfarbstoffe eine unterschiedliche Fluoreszenzabklingzeit aufweisen.

2. Verfahren nach Anspruch 1,
dadurch gekennzeichnet,
daß die zu erfassenden Moleküle Biomoleküle sind wie Nukleinsäure, Proteine, Peptide, Hormone, Antigene, Antikörper, Haptene oder toxische Substanzen, wie Umweltgifte, Pestizide sowie Polymere oder pharmazeutische Wirkstoffe, wie Alkaloide.

3. Verfahren nach Anspruch 1,
dadurch gekennzeichnet,
daß die Fluoreszenzfarbstoffe aus der Gruppe der Cumarine, Flavine,Fluoresceine, Rhodamine, Carbocyanine, Phenoxazone, Carbazine oder Oxazine gebildet werden.

4. Verfahren nach Anspruch 3,
dadurch gekennzeichnet,
daß die Fluoreszenzfarbstoffe aus Farbstoffen gebildet werden, die im fernen UV/VIS- bzw. nahen IR-Bereich absorbieren und fluoreszieren.

5. Verfahren nach Anspruch 1,
dadurch gekennzeichnet,
daß die Markierung mit Fluoreszenzfarbstoffen über eine kovalente Bindung zwischen Farbstoff und Molekül erfolgt.

6. Verfahren nach Anspruch 1,
dadurch gekennzeichnet,
daß die Markierung von Molekülen mit Farbstoffen spezifisch erfolgt.

## Claims

1. Process for the simultaneous, qualitative and quantitative determination by optical means of various molecules of a mixture, said molecules being marked with dyes or dye conjugates and excited to fluoresce by a laser system of light pulses of short duration, where the fluorescent light emitted is recorded and analysed with time resolution,
characterised in that,
dyes with specifically acting substituents on the same skeleton are used as fluorescent dyes, so that the fluorescent dyes have different fluorescence decay times.

2. Process according to claim 1,
characterised in that,
the molecules to be determined are biomolecules, such as nucleic acid, proteins, peptides, hormones, antigens, antibodies, haptens or toxic substances, such as environmental toxins, pesticides and polymers or pharmaceutical agents, such as alkaloids.

3. Process according to claim 1,
characterised in that,
the fluorescent dyes are made up of the group of coumarins, flavins, fluoresceines, rhodamines, carbocyanines, phenoxazones, carbazines or oxazines.

4. Process according to claim 3,
characterised in that,
the fluorescent dyes are formed from dyes which absorb and fluoresce in the far UV/visible or very near infra-red ranges.

5. Process according to claim 1,
characterised in that,
the marking with fluorescent dyes is effected by means of a covalent bond between dye and molecule.

6. Process according to claim 1,
characterised in that,
the marking of molecules with dyes is specific.

## Revendications

1. Procédé pour l'enregistrement simultané visuel, qualitatif et quantitatif de différentes molécules d'un mélange marquées par des matières colorantes ou des conjugués de matières colorantes, lesquelles molécules sont excitées dans un système laser par de brèves impulsions lumineuses pour fluorescer, la lumière fluorescente émise étant enregistrée et exploitée avec une résolution temporelle, **caractérisé en ce que** des matières colorantes avec des substituants agissant spécifiquement sont utilisées comme matière colorante fluorescente sur la même structure fondamentale de la matière colorante, les matières colorantes fluorescentes présentant une durée de déclin de fluorescence différente.

2. Procédé selon la revendication 1, **caractérisé en ce que** les molécules à enregistrer sont des biomolécules comme l'acide nucléïque, les protéines, les peptides, les hormones, les antigènes, les anticorps, les haptènes ou des substances toxiques comme les polluants de l'environnement, les pesticides, ainsi que des polymères ou des matières actives pharmaceutiques comme les alcaloïdes.

3. Procédé selon la revendication 1, **caractérisé en ce que** les matières colorantes fluorescentes sont formées à partit du groupe des coumarines, flavines, fluorescéines, rhodamines, carbocyanines, phénoxazones, carbazines ou oxazines.

4. Procédé selon la revendications 3, **caractérisé en ce que** les matières colorantes fluorescentes sont formées de colorants qui absorbent et fluorescent dans la zone à UV/VIS lointain ou dans la zone à infrarouges proches.

5. Procédé selon la revendication 1, **caractérisé en ce que** le marquage avec des colorants fluorescents s'effectue à l'aide d'une liaison covalente entre le colorant et la molécule.

6. Procédé selon la revendication 1, **caractérisé en ce que** le marquage de molécules avec des colorants est effectué de façon spécifique.
